# EUROPEAN PATENT SPECIFICATION

(11) **EP 0 848 936 B1**
(45) Date of publication and mention of the grant of the patent: **19.03.2003**
(21) Application number: 97122410.0
(22) Date of filing: 18.12.1997
(51) Int. Cl.: A61F 13/02

(54) **Method of manufacturing medicinal patch for percutaneous administration and apparatus for making the same**
Verfahren zur Herstellung von medizinischen Pflastern zur perkutanen Verabreichung, und Vorrichtung dafür
Méthode pour la fabrication de pansements médical pour administration percutanée, et appareil de fabrication

(30) Priority: 21.12.1996 KR 9669413
(43) Date of publication of application: 24.06.1998
(73) Proprietor: Sunkyong Industries Co., Suwon-si, Kyungki-do 440-745 (KR); Sunkyong Pharmaceuticals Ltd., Ansan-si, Kyungki-do 425-100 (KR)
(72) Inventor: Uhm, Ki Ahn, Suwon-si, Kyungki-do, Seoul 440-290 (KR); Min, Dong Sun, Seongnam-si, Kyeongki-do, 463-060 (KR)
(74) Representative: Koepe, Gerd L., Dipl.-Chem.

(56) References cited:
- EP-A- 0 571 700
- WO-A-94/21207
- US-A- 2 680 943

## Description

### BACKGROUND OF THE INVENTION

### 1. Field of the Invention

The present invention relates to a method of manufacturing medicinal patch for percutaneous administration and an apparatus for making the same. More particularly, it relates to a method of manufacturing transdermal skin patch which can fulfill its physical requirements, that is, it has such capability as to adhere to the skin of the knee or elbow securely and elastically for a long period of time even during bath or shower bath, and be not easily contaminated by the dirt without loss of medicinal properties.

The patch provided by the present invention can be accomplished by attaching a base film which contains medicinal properties over an adhesive tape through a series of manufacturing steps of the present invention. The base film which is used in making the patch of the invention has a structure of lamination of a separate liner, a medicament layer, a first elastic film layer and a first release liner in order; and the adhesive tape which is also used in making the patch of the invention has a structure of lamination of a second elastic film, an adhesive layer and a second release liner in order.

### 2. Discussion of Related Art

WO-A-94/21207 discloses a method of manufacture for a windowless frame delivered dressing.

It has been reported that piroxicam, a non-steroidal antiinflammatory and analgesic agent, shows competent efficacy against acute pain due to rheumatoid arthritis, osteoarthritis, and gout etc. when applied by oral administration but causes unfavorable side effects such as gastroenteric disorder, gastric or duodenal ulcer, and the like. In this sense, it is considerably advisable to apply piroxicam by other than oral administration, that is, such as by percutaneous administration.

In the medicinal patch for percutaneous administration, the preparations of its medicinal substrate are of course a matter of primary importance, and the physical characteristics of the patch are by no means the least importance if we are to achieve the desired efficacy of drug over the percutaneous administration. That is, aparting from what the composition of the medicinal substrate contained in the patch is, the patch must securely adhere to an articulation such as the knee or elbow maintaining its elasticity even during bath or shower bath, and should be free from contamination and loss of medicinal properties. Further, the medicinal patch preferably has a round or oval shape and is thin and small so as to facilitate topical application thereof. However, conventional medicinal patches in which medicaments are simply applied over a non-woven fabric or textile fail to meet the above requirements.

### SUMMARY OF THE INVENTION

Accordingly, the present invention is directed to provide a method and apparatus of manufacturing medicinal patch for percutaneous administration that is thin and small and is of oval or round shape to be securely and elastically adhered to the skin of the human body for a long period of time without loss of properties.

To achieve these and other advantages and in accordance with the purpose of the present invention, as embodied and broadly described, there is disclosed a method of manufacturing a medicinal patch for percutaneous administration comprising the following steps :
continuously unwinding a base film which has the structure of a laminate of a separate liner, a medicament layer, a first elastic film layer and a first release liner in order from one of first feed rollers, while removing said first release liner from said base film;
performing a continuous first half-die cutting only on the separate liner by passing the released base film between a pair of first half-die cutting rollers, so that at least one wave-shaped cut-and-tear line is lengthwise formed on the separate liner;
performing a continuous second half-die cutting only on the medicament layer and the first elastic film layer disposed on the separate liner into a predetermined shape and, then, removing a scrap of the medicament layer and the first elastic film layer from the separate liner, whereby on the separate liner remain the processed medicament layer and the first elastic film layer in the predetermined shape to make a first resultant strip;
continuously unwinding an adhesive tape which has the structure of a laminate of a second elastic film layer, an adhesive layer and a second release liner in order from a second feed roller, while removing the second release liner from said adhesive tape and, then, attaching said released adhesive tape over said first resultant strip;
performing a continuous third half-die cutting only on the released adhesive tape which is attached to the first resultant strip into a predetermined shape at the corresponding regions to those of said second half die-cutting, while removing a scrap of said adhesive tape produced by the third half-die cutting, whereby on the separate liner remain the processed medicament layer, the first elastic film layer, the adhesive layer and the second elastic film layer in the predetermined shape to make a second resultant strip;
performing a continuous die cutting on said second resultant strip into a predetermined shape at the corresponding regions to those of said half-die cuttings, while removing a scrap of separate liner created by said die cutting, thus, producing finished products of medicinal patch; and
drawing up the finished products of medicinal patch from the main die roller by a vacuum pad roller and transferring them to a conveyor.

Another aspect of the present invention, an apparatus for manufacturing a medicinal patch for percutaneous administration comprises :
first upper and lower feed rollers, any one of said first feed rollers unwinding and feeding a base film whose structure is a laminate of a separate liner, a medicament layer, a first elastic film layer and a first release liner in order;
a first release liner winding roller which winds the first release liner therearound for removal;
a pair of first half-die cutting rollers performing a first half-die cutting just on the separate liner of the base film to form at least one wave-shaped cut-and-tear line lengthwise thereon;
a second half-die cutting roller with a plurality of oval- or round-shaped half-die cutting blades equidistantly installed on its outer surface, performing a second half-die cutting on each of the medicament layer and the first elastic film layer disposed on the separate liner of the base film, into an oval or round shape;
a scrap winding roller around which a scrap of the medicament and first elastic film layers produced by the second half-die cutting, is wound for removal;
a second feed roller unwinding an adhesive tape whose structure is a laminate of a second elastic film layer, an adhesive layer and a second release liner in order;
a second release liner winding roller around which the second release liner is wound for removal;
a pair of pressure rollers attaching a released adhesive tape laminated the base film with the medicament and first elastic film layers half-die cut;
a third half-die cutting roller with a plurality of oval-or round-shaped half-die cutting blades equidistantly installed on its outer surface, performing a third half-die cutting on regions of the adhesive tape attached to the base film into an oval or round shape at the corresponding regions to those of said second half-die cutting;
a roller on which a scrap of the adhesive tape produced by the third half-die cutting is wound for removal;
a die cutting roller with a plurality of die cutting blades equidistantly installed on its outer surface, performing a continuous die cutting of a resultant strip which has the separate liner, the ovally or circularly punched medicament and first elastic film layers, the ovally or circularly punched adhesive and second elastic film layers, into such a predetermined shape as enclosing regions formed through the second and third half-die cutting steps;
a roller on which a scrap of the resultant strip created by the die cutting is wound for removal;
a main die roller having a plurality of vacuum holes on its outer surface for drawing up the separate liner of the base film, and performing the second half-die cutting on each of the medicament layer and the first elastic film layer formed on the separate liner of the base film with the help of said second half-die cutting roller, the third half-die cutting on the released adhesive tape with the help of said third half-die cutting roller and the die cutting on the overall resultant strip wich has the separate liner, the ovally or circularly punched medicament and first elastic film layers and the ovally or circularly punched adhesive and second elastic film layers with the help of said die cutting roller, thus, producing finished products of medicinal patch; and
a vacuum pad roller having a plurality of rubber sucking means on its outer surface for drawing up the finished products from the main die roller and transferring them to a conveyor.

It is to be understood that both the foregoing general description and the following detailed description are exemplary and explanatory and are intended to provide further explanation of the invention as claimed.

### BRIEF DESCRIPTION OF THE ATTACHED DRAWINGS

Figs. 1(a) and 1(b) are each a plan view of a medicinal patch provided in accordance with the present invention, and an enlarged-sectional view thereof as taken along the line a - a of Fig. 1(a).

Fig. 2(a) shows a perspective view of a base film roll used for making the inventive medicinal patch for percutaneous administration, and a sectional view thereof as taken along the line b - b.

Fig. 2(b) shows a perspective view of an adhesive tape roll used for the inventive medicinal patch for percutaneous administration, and a sectional view thereof as taken along the line c - c.

Fig. 3 schematically depicts steps of manufacturing the medicinal patch for percutaneous administration in accordance with the present invention.

Figs. 4(a) to 4(m) are each plan and sectional views of respective base film strip and/or adhesive tape strip or its scrap to be removed according to the steps of manufacture the medicinal patch in accordance with the present invention.

Fig. 5 is a front view of an apparatus for manufacturing medicinal patch for percutaneous administration in accordance with the present invention.

### DETAILED DESCRIPTION OF PREFERRED EMBODIMENT

Reference will now be made in detail to the preferred embodiments of the present invention, examples of which are illustrated in the accompanying drawings. Like reference numerals denote like reference parts throughout the specification and the drawings.

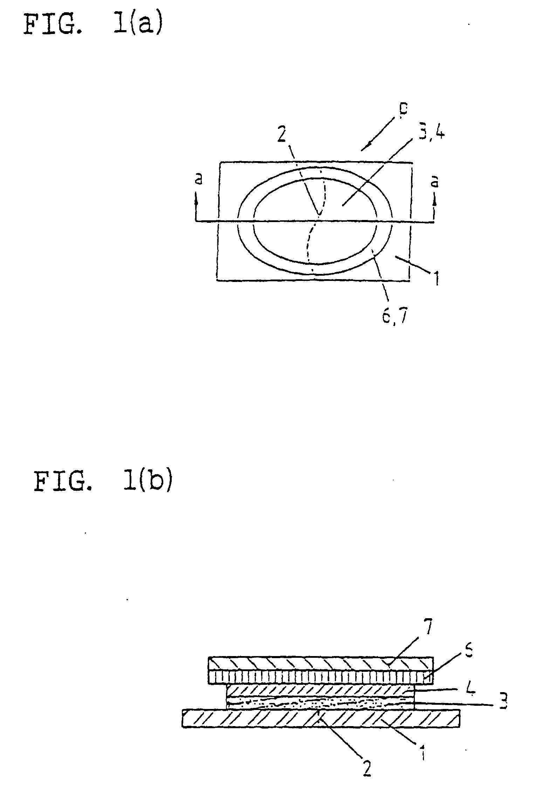

A preferable medicinal patch which obviates the drawbacks of the conventional art and fulfills the physical requirement of a transdermal skin patch is depicted in Figs. 1(a) and 1(b).

Fig. 1(a) is a plan view of the medicinal patch provided according to the present invention, and Fig. 1(b) is an enlarged-sectional view thereof as taken along the line a - a of Fig. 1(a). Reference numeral 1 designates a separate liner that is to be finally removed when the patch P is applied to the skin of the human body. The separate liner 1, being used as a releaser at the time of use, is favorably formed by coating silicon on such the resin as polyethylene terephthalate (PET) or paper. As can be seen from Fig. 1(b) which shows a section of the patch, on the separate liner 1 are deposited a medicament layer 3, a first elastic film layer 4, an adhesive layer 6 and a second elastic film layer 7 in order. The first elastic film layer 4 has an elongation of more than about 100% and thickness of 5 to 300 micrometers and is preferably made from polyurethane (PU), polyethylene terephthalate (PET) or styrene iso-styrene (SIS), and the like.

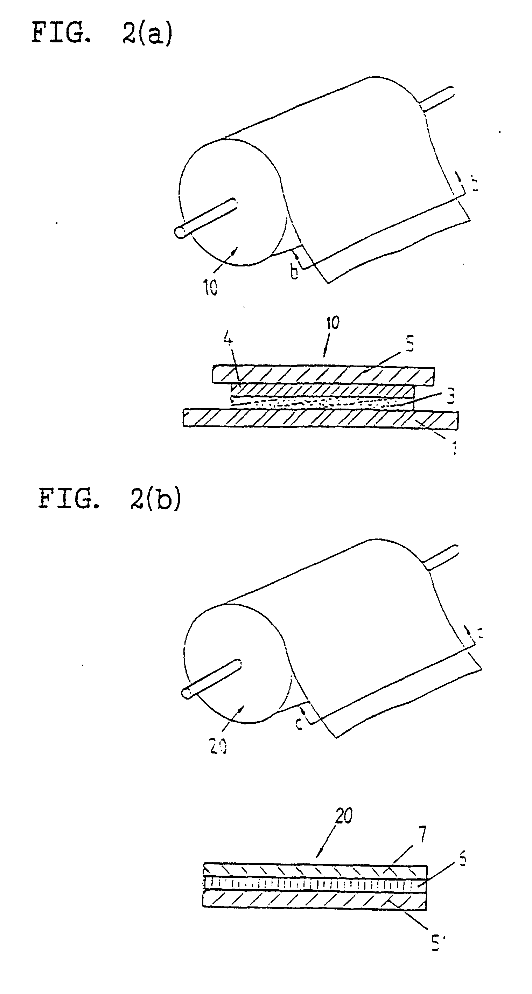

A base film 10 which is used as one of main stuffs in making the patch P of the invention is shown in Fig. 2(a) and has a structure of lamination of a separate liner 1, a medicament layer 3, a first elastic film layer 4 and a first release liner 5 in order. A adhesive tape 20 which is also used as another main stuff in making the patch of the invention has a structure of lamination of a second release liner 5', an adhesive layer 6 and a second elastic film 7 in order as can be seen in Fig. 2(b). The base film 10 and the adhesive tape 20 undergo a series of processing steps such as a few times half-die cutting, mutual lamination, die cutting and drawing-up etc. with the help of the apparatus of the present invention.

A method of manufacturing the base film 10 has been fully described in another korean patent application filed by us on the same date of priority of the present invention, entitled "Method of manufacturing a base film for a transdermal skin patch and an apparatus for making the same". Since the method of manufacturing the base film 10 itself is another invention and not material to understand the present invention, the detailed description thereabout is omitted in this specification. Furthermore, the description about a method of manufacturing the adhesive tape 20 itself is also omitted for the same reason.

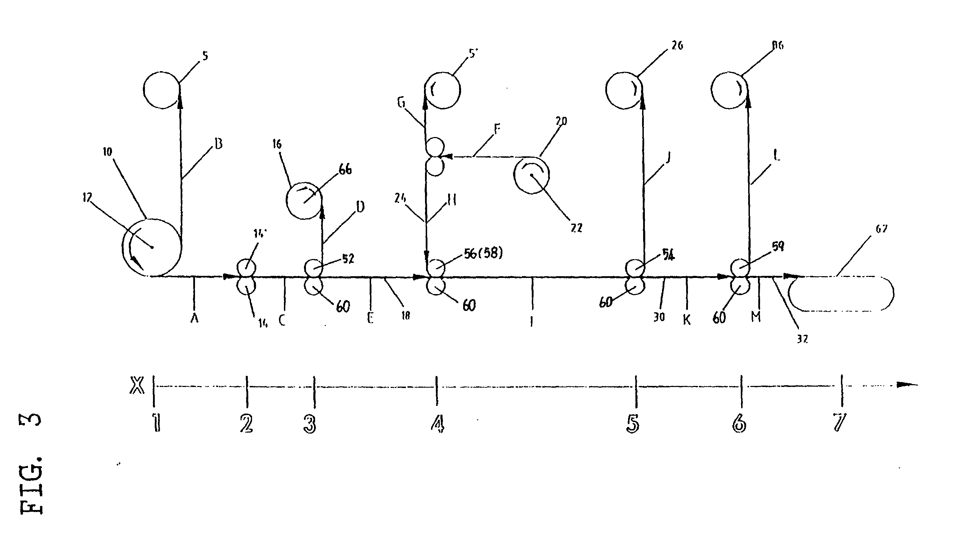

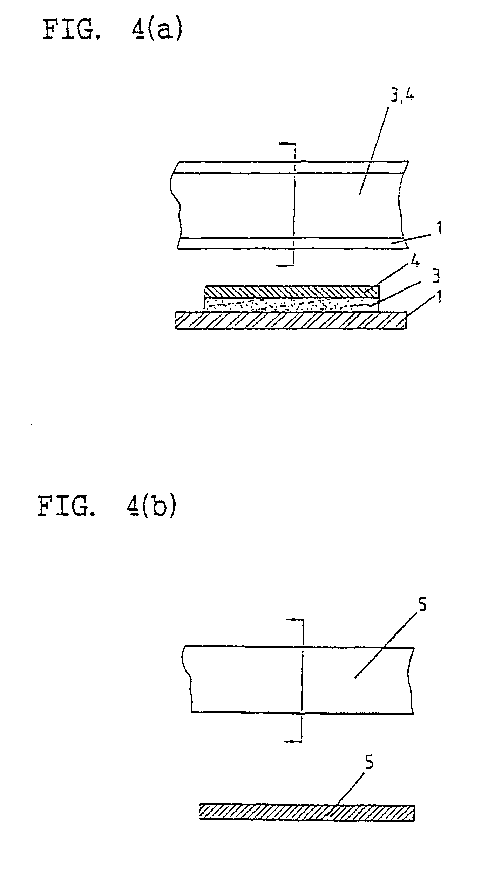

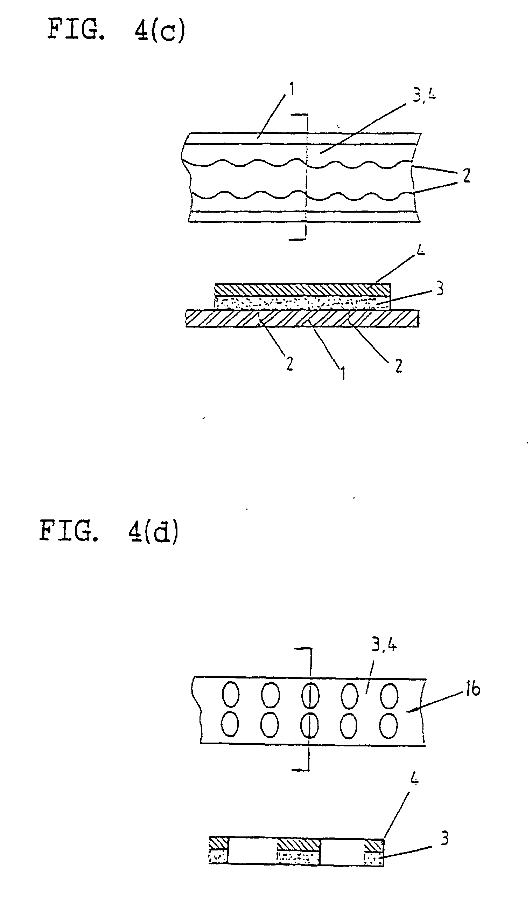

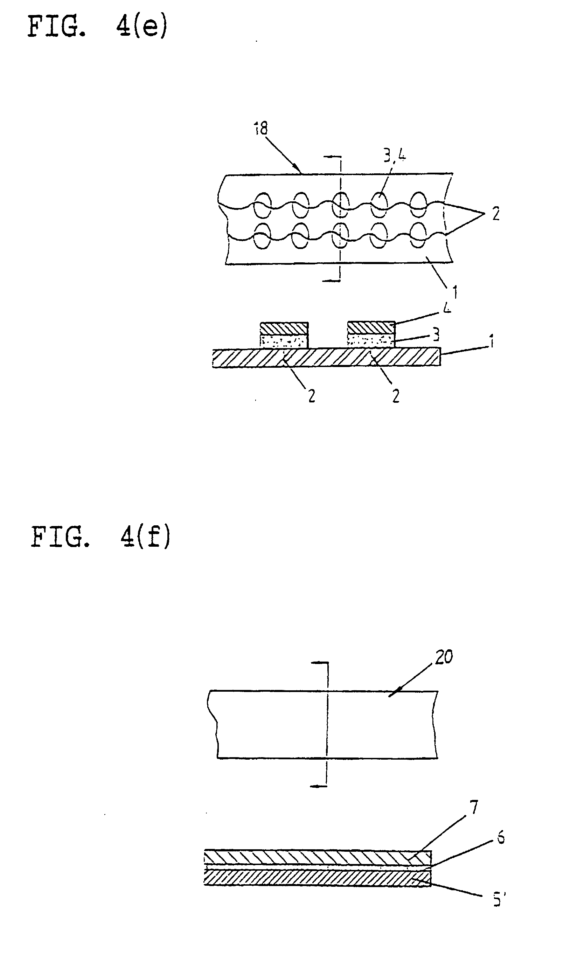

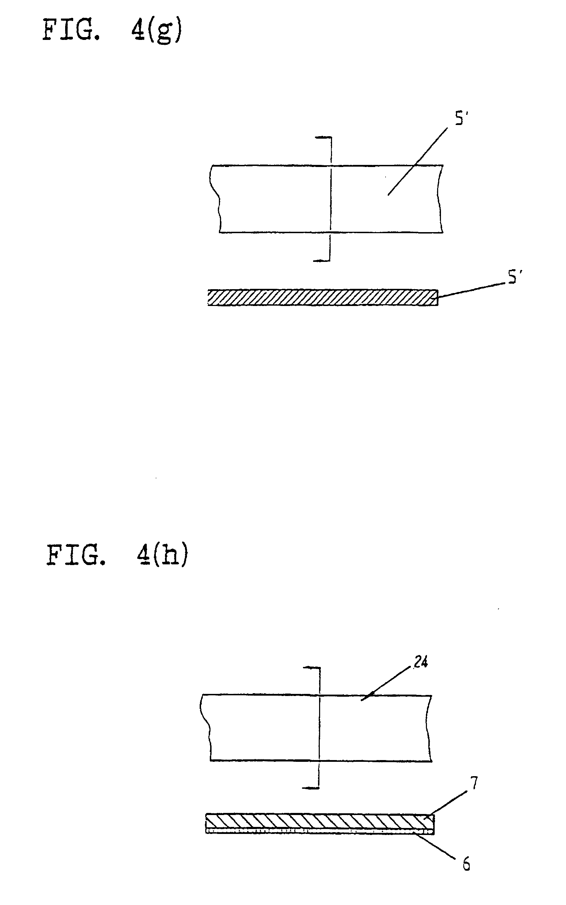

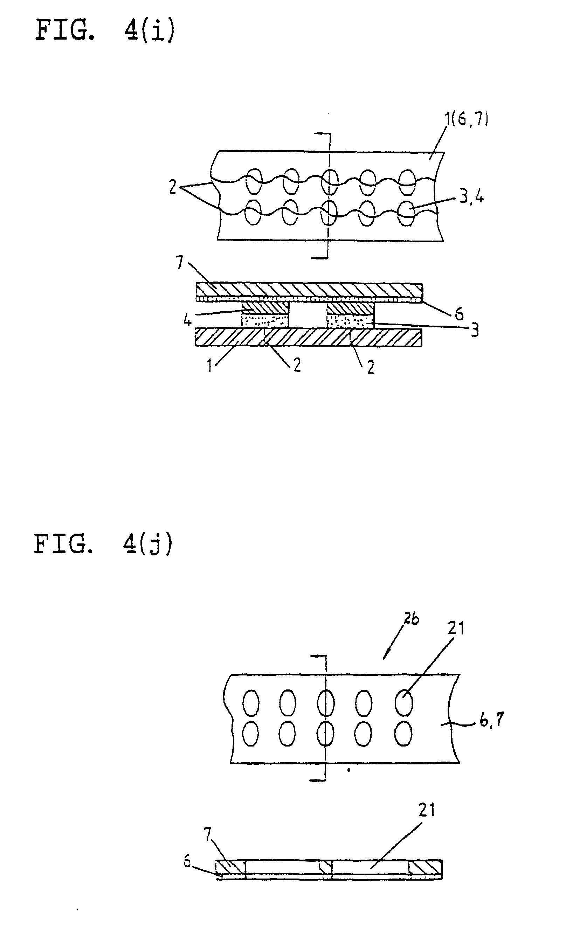

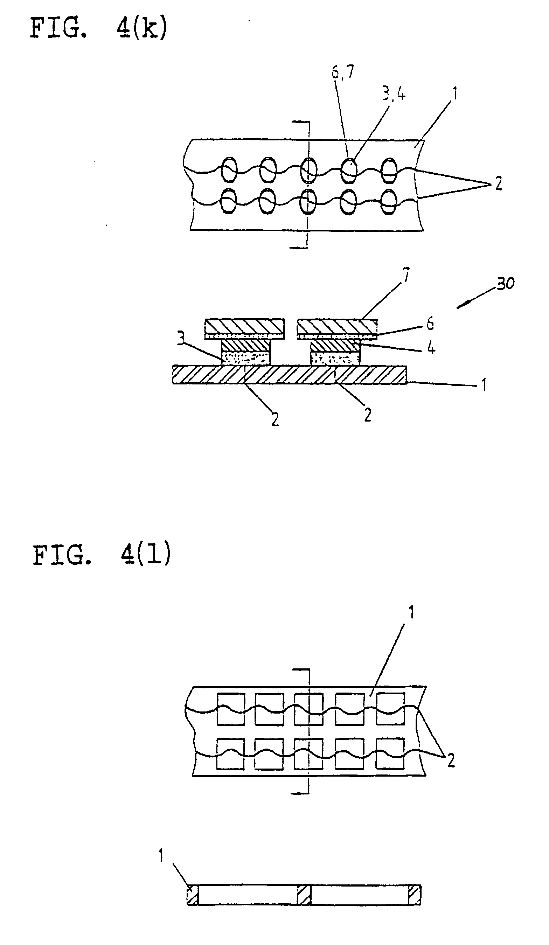

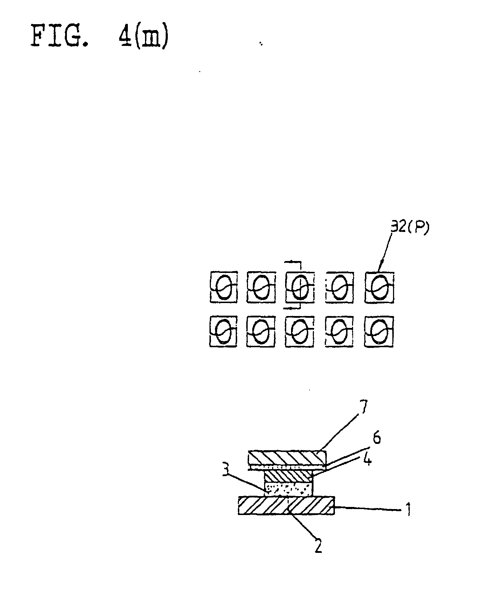

Fig. 3 schematically depicts the steps of manufacturing the medicinal patch for percutaneous administration in accordance with the present invention and Figs. 4(a) to 4(m) are each plan and sectional views of respective base film strip and adhesive tape strip or its/their residual scrap/scraps to be removed in the process of the present invention.

Referring to Figs. 3 to 4(m), the steps of manufacturing the inventive medicinal patch are now fully described. Axis X of Fig. 3 indicates the development of the manufacturing steps in accordance with the present invention.

### FIRST STEP

The base film 10 which is provided with a form of roll and whose structure is a lamination of the separate liner 1, the medicament layer 3, the first elastic film layer 4 and the first release liner 5 in order is drawn out from one of first feed rollers 12 and 12' while the first release liner 5 is peeled off from the base film 10 for removal and wound around its winding roller 60 (Hereinafter the base film with no release liner 5 being preferably called 'released base film'). This step can be referred to Figs. 3, 4(a) and 4(b).

### SECOND STEP

The released base film 10, being unwound from one of first feed rollers 12 and 12', passes through a pair of first half-die cutting rollers 14 and 14' so that at least one wave-shaped cut-and-tear line 2 is consecutively formed only on the separate liner 1 along the length of the released base film 10 as depicted in Fig. 3 and 4(c).

In the medicinal patch for percutaneous administration manufactured by using the inventive apparatus, each of the medicament layer 3 and the elastic film layers 4 and 7 is no more than a few tens of micrometers in thickness and the overall medicinal patch P is so thin that it is not easy to remove the separate liner 1 from the patch unless appropriate cut-and-tear line is provided therein when applying the patch to the diseased region of the body. Therefore, the second step is carried out for consecutively forming at least one cut-and-tear line 2 just on the separate liner 1 by the half-die cutting technique.

### THIRD STEP

The medicament layer 3 and the first elastic film layer 4, overlapped on the separate liner 1 of the base film 10, are simultaneously half-die cut into an oval or round shape on a main die roller 60 through second half-die cutting. A scrap 16 of the medicament layer 3 and the first elastic film layer 4 created from the second half-die cutting is wound on a roller 66 to be removed from the separate liner 1, as shown in Figs. 3 and 4(d) and 4(e).

A first resultant strip 18 where the medicament layer 3 and the first elastic film layer 4 are remained in the shape of oval or circle on the separate liner 1 by said second half-die cutting and its consequent removal of the scrap 16, is transferred forward while maintaining close contact to the outer surface of the main vacuum die roller 60.

### FOURTH STEP

The adhesive tape 20 which is prepared as a roll and whose structure is a lamination of the second release liner 5', the adhesive layer 6 and second elastic film layer 7 is continuously provided from a second feed roller 22 while its second release liner 5' being removed therefrom and collected on its winding roller 70 and, thus, makes a released adhesive tape 24 with no release liner 5'. After being peeled off the second release liner 5', the released adhesive tape 24 is then attached over the first resultant strip 18 where the medicament layer 3 and the first elastic film layer 4 are remained in the shape of oval or circle on the separate liner 1, which is well depicted in Figs. 3 and 4(f), 4(g), 4(h) and 4(i).

The fourth step is also carried out on the main die roller 60 with the help of pressing rollers 56 and 58 which give attaching pressure to the first resultant strip 18 against the main die roller 60 right after the released adhesive tape strip 24 is laid over the first resultant strip 18.

### FIFTH STEP

From this step, the released adhesive tape 24 which has been attached over the first resultant strip 18 are manipulated to have the coincidental oval or circular shape just on the corresponding oval or circular medicament layer 3 and the first elastic film layer 4 by a third half-die cutting. However, it should be noted that the oval or circular regions of the adhesive tape 24 have the same shapes as those of the medicament layer 3 and the first elastic film layer 4 of the first resultant strip 18 but are a little larger than the medicament layer 3 and the first elastic film layer 4 so as to let the differentiated larger area have a role of adhering capability to the skin. A scrap 26 of the released adhesive tape produced at this point is removed by being wound on a roller 74, thus, creating a second resultant strip 30. This process can be well referred to in Figs. 3, 4(j) and 4(k).

The second resultant strip 30 which has the consecutive separate liner 1 with at least one cut-and-tear line 2 therein, the ovally or circularly shaped medicament layer 3 and first elastic film layer 4 lied on said separated liner 1 and the ovally or circularly shaped adhesive layer 6 and second elastic film layer 7 lied just on said ovally or circularly shaped medicament layer 3 and first elastic film layer 4 is drawn up and transferred forward.

### SIXTH STEP

Referring to Fig. 4(l), a die cutting is performed on the second resultant strip 30 into a predetermined shape (e.g. square shape), thus, producing a third resultant strip 32. At this stage, a scrap 86 of the separate liner 1 created from this die cutting is finally removed. The third resultant strips 32 make finished products of medicinal patch prior to packing for sale.

### SEVENTH STEP

The finished products of the third resultant strip 32 are drawn up and transferred from the main die roller 60 to a conveyor 62 by a vacuum pad roller 90.

The method of making the medicinal patch for percutaneous administration comprises the above-described seven steps.

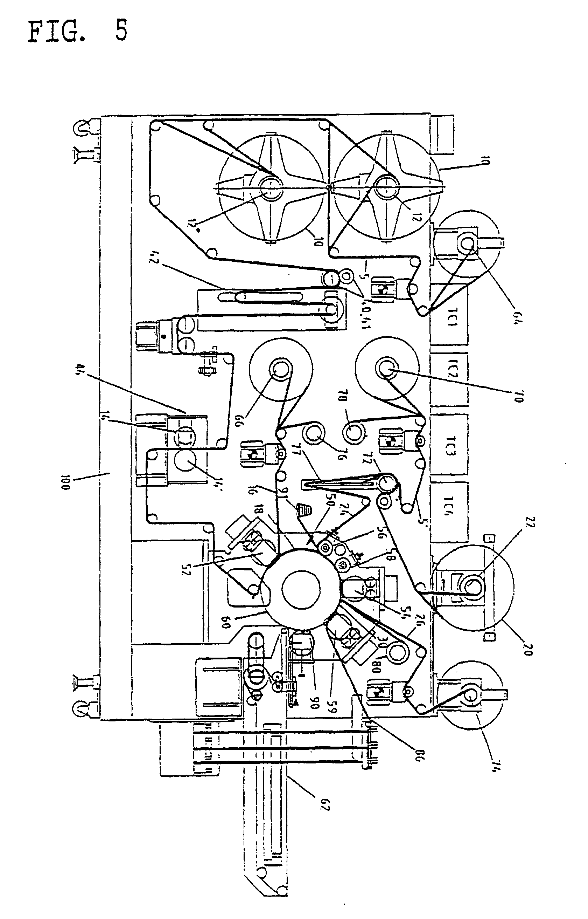

Fig. 5 is a front view of an apparatus for performing said manufacturing steps for manufacturing medicinal patch for percutaneous administration in accordance with the present invention.

Reference numeral 100 denotes a frame of the apparatus for manufacturing the inventive medicinal patch P which is movable with a jack or jacks(not drawn), and made preferably from stainless steel. The first feed rollers 12 and 12' for mounting the roll of base film 10 and drawing it out are rollingly mounted on the frame up and down, so one of them unwinds the base film 10 selectively. If the base film roll mounted on any one of the upper and lower rollers 12 and 12' is all run out, a spare base film roll provided on the other feed roller starts to be drawn out without delay, which saves the loading time to enhance the productivity. These rollers 12 and 12' include a controller to minutely control the base film roll's mounting condition, a hand brake, a device for alarming decrement of the base film, and a sensor for detecting the cutting thereof(not shown).

A pair of rollers 40 and 41 which are so provided as to transfer the base film 10 control their rolling speed according to displacement of a dancer roller 42 having a balancer, and assures the constant-speed transfer of the base film. The dancer roller 42 keeps a predetermined weight by the balancer, and transfers the base film 10 to the main die roller 60 under a given tension regardless of the remained diameter of the base film roll wound around the feed roller 12 or 12'.

A cutting apparatus 44 which comprises a pair of the first die cutting rollers 14, 14' serves to half-die cut only the separate liner 1 of the base film 10 into a waveform. As the base film 10 passes between a pair of the first half-die cutting rollers 14 and 14' (first half-die cutting rollers), the separate liner 1 is half-die cut to have at least one continuous wave-shaped line 2 along its longitudinal direction.

Reference numeral 50 denotes a main unit of the apparatus for making the inventive medicinal patch. By the main unit 50 are performed the following five steps: one is the second half-die cutting by which the medicament layer 3 and the first elastic film layer 4 which are laminated over the separated liner 1 are processed into an oval or round shape; another is attaching the released adhesive tape 24 to the first resultant strip 18; the next is is the third half-die cutting the adhesive tape into an oval or round shape; the next is performing a die cutting the resultant strip 32 to a predetermined shape (e.g. square shape) to produce finished products of medicinal patch P and the last is finally drawing up the finished products by the vacuum pad roller 90 and moving them to the conveyor 62.

A second half-die cutting roller 52 for half-die cutting only the medicament layer 3 and the first elastic film layer 4 into an oval or round shape, and a third half-die cutting roller 54 for half-die cutting only the adhesive tape 24 into an oval or round shape, each have a plurality of oval or round-shaped kiss cutting blades equidistantly formed along their outer surfaces. A die cutting roller 59 also has a plurality of square-shaped kiss cutting blades formed equidistant from one another at its outer surface.

A plurality of vacuum holes(not drawn) are formed on the outer surface of the main die roller 60 for drawing up and transferring the base film 10 during the second, third half-die cutting steps and final die cutting step.

The preliminary and main pressure rollers 56 and 58 used for adhering the adhesive tape 24 over the first resultant strip 18 are interposed between the second half-die cutting roller 52 and the third half-die cutting roller 54. The preliminary pressure roller 56 is an auxiliary one, and the roller 58 is a main one. The process of rolling and compressing the released adhesive tape strip 24 against the first resultant strip 18 on the main die roller 60 prevents bonding failure(for example, air gap entrapped between the base film and the adhesive tape).

The vacuum pad roller 90 has rubber suckers(not drawn) on its outer surface for drawing up the finished products of medicinal patch from the surface of the main die roller 60 by suction, and transfers them to the conveyor 62.

During performing the process described in the above, the first release liner 5 of the base film 10 is collected on a first release liner winding roller 64 under a constant tension with the help of a powder clutch(not drawn) and a tension controller (TC1) regardless its winding diameter.

Reference numeral 66 denotes a roller on which a scrap 16 of the medicament layer 3 and the first elastic film layer 4 is wound. The scrap 16 is wound on the first release liner winding roller 66 under a constant tension with the help of a powder clutch(not drawn) and a tension controller (TC2) regardless of the diameter of the scrap 16 wound.

The adhesive tape roll 20 is the second feed roller 22. Reference numeral 70 is a second release liner winding roller with a single-end fixing type shaft on which the second release liner 5' is collected after being separated from the adhesive tape 20.

An alarming system is provided to the second release liner winding roller 70 for informing an operator of the second release liner 5's being fully wound thereon. The winding tension is constantly kept by a powder clutch(not drawn) and a tension controller (TC3) when winding the release liner 5'.

Reference numeral 74 denotes a roller on which the scrap 26 of the adhesive tape. produced after the third half-die cutting, is wound. An alarming system is also provided to the roller 74, thus informing the operator of the scrap 26's being fully wound on the roller 74. The tension of the strap 26 is constantly maintained by a powder clutch (not drawn)and a tension controller (TC4) when winding the scrap 26 on the roller 74.

A peeling roller 72 has a plurality of vacuum holes(not drawn) on its outer surface for drawing up the adhesive tape strip while separating the second release liner 5' from the adhesive tape strip 20. The adhesive tape strip, from which the second release liner 5' is separated by the peeling roller 72, becomes free from its beared tension by a dancer roller 77 having a given weight kept by a balancer, and avoids its gradual shrinking due to residual tensile stress when the released adhesive tape strip 24 is attached to the first resultant strip 18.

Reference numerals 76, 78, and 80 designate temporarily winding rollers, and these are positioned adjacent to the scrap winding roller 66, the second release liner winding roller 70, and the roller 74 on which the scrap 26 of the adhesive tape is wound, respectively. The rollers 76, 78, and 80 are used only when the scraps are fully wound on the rollers 66, 70, and 74, respectively and so the used ones are to be replaced with the new empty ones. All the rollers 76, 78, and 80 have a same role to save loading or replacing time. These rollers 76, 78, and 80 are preferably configured for non-viscidity to prevent the scraps from sticking to the outer surface of each of them.

Ovally or circularly punched portions 21 are serially formed on the scrap 26 of the released adhesive tape 24 by the third half-die cutting, as shown in Fig. 4(j), and in particular, its tensile strength becomes considerably lessen so that the scrap 26 is easily broken during its winding. Once the scrap 26 is broken, the apparatus cannot perform the next steps, thus lowering the productivity.

In connection with this, reference numeral 91 denotes a means for increasing the tensile strength of the scrap 26 and preventing cutting of the scrap 26 during winding after the adhesive tape 24 is adhered to the first resultant strip 18. Preferably, the tensile stress reinforcing means is realized by thread or string provided to regions of the both longitudinal edges and/or middle portion of the punched scrap 26 of the adhesive tape 24.

The following description relates to the operation of the apparatus for making the inventive medicinal patch P.

The base film 10, fed from one of the first feed rollers 12 and 12' at a required constant speed by a pair of transferring rollers 40 and 41, passes between the first half-die cutting rollers 14 and 14' so that only the separate liner 1 of the base film 10 is continuously half-die cut to have at least one wave-shaped cutting line 2(the first half-die cutting).

After the first half-die cutting, the released base film travels the outer surface of the main die roller 60 by closely vacuum contact thereon in accordance with the rolling movement of the main die roller and, then, the medicament layer 3 and the first elastic film layer 4 of the base film 10 are cut out by the oval or round kiss cutting blades equidistantly formed along the outer surface of the second half-die cutting roller 52.

The adhesive tape 24 produced from the adhesive tape roll 20 by peeling off the second release liner 5' therefrom is attached over the first resultant strip 18 with the help of the pressing rollers 56 and 58. In order to give more tensile stress to the scrap 26 of the adhesive tape 24, the thread or string 91 is provided to the back of the adhesive tape 24.

As the second resultant strip 30 formed by attaching the adhesive tape 24 to the first resultant strip 18 is moved a little way along the main die roller 60, the third half-die cutting roller 54, having a plurality of oval- or round-shaped kiss cutting blades equidistantly installed on its outer surface, half-die cuts only the adhesive tape 24 of the second resultant strip into an oval or round shape. A scrap 26 produced from the above process is wound on the roller 74 for removal. At this stage, the third half-die cutting is carried out on the regions of the adhesive tape 24 corresponding to the ovally or circularly shaped medicament and first elastic film layers 3 and 4.

The strip on which the third half-die cutting is performed is moved a little way along the main die roller 60 closely contacting the main die roller 60. The overall strip is then die cut out by the die cutting roller 59 which has a plurality of square-shaped kiss cutting blades formed equidistant from one another at its outer surface, thus producing finished products of medicinal patch P as shown in Fig. 1. The vacuum pad roller 90 draws up each finished product with its rubber suckers that are circumferentially formed equidistantly from each other, and transfers it to the conveyor 62. Automatic inspection and packing are performed on each finished product P of medicinal patch on the conveyor 62.

As described above, medicinal patch for percutaneous administration made by the apparatus according to the manufacturing steps of the present invention can fulfill the physical requirements of a transdermal skin patch. That is, the inventive medicinal patch can adhere to the skin of the knee or elbow securely and elastically for a long period of time even during bath or shower bath, and is not easily contaminated by the dirt without loss of medicinal properties. In addition, the oval- or round-shaped medicinal patch of the present invention is so thin and small that it can be securely attached to the skin of any diseased region of the human body, thus being superior to the presently-available transdermal skin patch.

Additional advantages and modifications will readily occur to those skilled in the art. The invention in its broader aspects is not limited to the specific details, and representative devices, shown and described herein. Accordingly, various modifications may be made without departing from the scope of the general inventive concept as defined by the appended claims and their equivalents.

## Claims

1. A method of manufacturing a medicinal patch for percutaneous administration comprising the steps of:
continuously unwinding a base film which is a laminate of a separate liner, a medicament layer, a first elastic film layer and a first release liner in order from one of first feed rollers, while removing said first release liner from said base film;
performing a continuous first half-die cutting only on the separate liner by passing the released base film between a pair of first half-die cutting rollers, so that at least one wave-shaped cut-and-tear line is lengthwise formed on the separate liner;
performing a continuous second half-die cutting only on the medicament layer and the first elastic film layer disposed on the separate liner into a predetermined shape and, then, removing a scrap of the medicament layer and the first elastic film layer from the separate liner, whereby on the separate liner remain the processed medicament layer and the first elastic film layer in the predetermined shape to make a first resultant strip;
continuously unwinding an adhesive tape which is a laminate of a second elastic film layer, an adhesive layer and a second release liner in order from a second feed roller, while removing the second release liner from said adhesive tape and, then, attaching said released adhesive tape over said first resultant strip;
performing a continuous third half-die cutting only on the released adhesive tape which is attached to the first resultant strip into a predetermined shape at the corresponding regions to those of said second half die-cutting, while removing a scrap of said adhesive tape produced by the third half-die cutting, whereby on the separate liner remain the processed medicament layer, the first elastic film layer, the adhesive layer and the second elastic film layer in the predetermined shape to make a second resultant strip;
performing a continuous die cutting on said second resultant strip into a predetermined shape at the corresponding regions to those of said half-die cuttings, while removing a scrap of separate liner created by said die cutting, thus, producing finished products of medicinal patch; and
drawing up the finished products of medicinal patch from the main die roller by a vacuum pad roller and transferring them to a conveyor.

2. A method according to claim 1, wherein the second half-die cutting forms the medicament layer and the first elastic film layer into an oval or round shape, and the third half-die cutting forms the corresponding regions of the adhesive tape into an oval or round shape slightly larger than that of the second half-die cutting, while each punched regions of the resultant strips being formed by the die cutting so as to enclose each the second and third half-die cutting regions.

3. A method according to claim 1, wherein the step of attaching the released adhesive tape laminated to the first resultant strip is carried out by preliminary and main pressure rollers by two stages.

4. A method according to any one of claims 1 to 3, wherein when the released adhesive tape is attached to the first resultant strip, a thread or string is also attached to the back of the adhesive tape for adding more tensile strength to the scrap of the adhesive tape during its winding for removal.

5. An apparatus for manufacturing a medicinal patch for percutaneous administration comprising:
first upper and lower feed rollers, any one of said feed rollers unwinding and feeding a base film whose structure is a laminate of a separate liner, a medicament layer, a first elastic film layer and a first release liner in order;
a first release liner winding roller which winds the first release liner therearound for removal;
a pair of first half-die cutting rollers performing a continuous first half-die cutting just on the separate liner of the base film to form at least one wave-shaped cut-and-tear line lengthwise on the separate liner;
a second half-die cutting roller with a plurality of oval- or round-shaped half-die cutting blades equidistantly installed on its outer surface, performing a continuous second half-die cutting on each of the medicament layer and the first elastic film layer disposed on the separate liner of the base film, into an oval or round shape;
a scrap winding roller around which a scrap of the medicament and first elastic film layers produced by the second half-die cutting is wound for removal;
a second feed roller unwinding an adhesive tape whose structure is a laminate of a second elastic film layer, an adhesive layer and a second release liner;
a second release liner winding roller around which the second release liner is wound for removal;
a pair of pressure rollers attaching the released adhesive tape laminated to the first resultant strip;
a third half-die cutting roller with a plurality of oval-or round-shaped half-die cutting blades equidistantly installed on its outer surface, performing a continuous third half-die cutting on regions of the adhesive tape attached to the first resultant strip into an oval or round shape, corresponding to the second half-die cut regions of the medicament and first elastic film layers;
a roller on which a scrap of the adhesive tape produced by the third half-die cutting is wound for removal;
a die cutting roller with a plurality of die cutting blades equidistantly installed on its outer surface, performing a continuous die cutting of a second resultant strip which has the separate liner, the ovally or circularly punched medicament and first elastic film layers, the ovally or circularly punched adhesive and second elastic film layers, into a predetermined shape, thus enclosing each region punched through the second and third half-die cutting steps;
a roller on which a scrap of the resultant strip, created by the die cutting, is wound for removal;
a main die roller having a plurality of vacuum holes on its outer surface for drawing up the separate liner of the base film, and performing the second half-die cutting on the medicament layer and the first elastic film layer formed on the separate liner with the help of said second half-die cutting roller, the third half-die cutting on the released adhesive tape with the help of said third half-die cutting roller, and the die cutting on the second resultant strip with the help of said die cutting roller, thus, producing finished products of medicinal patch; and
a vacuum pad roller having a plurality of rubber sucking means on its outer surface for drawing up the finished products from the main die roller and transferring them to a conveyor.

6. An apparatus according to claim 5, wherein a pair of the pressure rollers are interposed between the second and third half-die cutting rollers and one of them is a preliminary pressure roller and the other is a main pressure roller.

7. An apparatus according to claim 5 or 6, further comprising means for adding more tensile strength to the scrap of the adhesive tape that is removed after the third half-die cutting.

8. An apparatus according to claim 7, wherein said means is a thread or string lengthwise provided to both longitudinal edges and /or middle portion of the adhesive tape.

9. An apparatus according to claim 5, further comprising temporarily winding rollers, each of which being adjacent to said medicament scrap winding roller, said second release liner winding roller, and said roller to wind the scrap of the adhesive tape respectively and used temporarily only when the scraps are fully wound on said three rollers.

## Patentansprüche

1. Verfahren zur Herstellung eines medizinischen Pflasters zur perkutanen Verabreichung, umfassend die Schritte, daß man
- einen Grund-Film, der ein Laminat aus einem getrennten Liner, einer ein Medikament umfassenden Schicht, einer Schicht aus einem ersten elastischen Film und einem Schutz-Liner in dieser Reihenfolge ist, kontinuierlich von einer der ersten Zufuhr-Walzen abrollt, während man den ersten Schutz-Liner von dem Grund-Film entfernt;
- einen ersten kontinuierlichen Halb-Stanz-Schritt nur an dem getrennten Liner durchführt, indem man den freigemachten Grund-Film zwischen einem Paar erster Halb-Stanz-Walzen hindurchtreten läßt, so daß wenigstens eine wellenförmige Schneid-Reiß-Linie in Längsrichtung auf dem getrennten Liner gebildet wird;
- einen zweiten kontinuierlichen Halb-Stanz-Schritt nur an der ein Medikament umfassenden Schicht und der Schicht aus dem ersten elastischen Film, die auf dem getrennten Liner angeordnet sind, zu einer vorbestimmten Form durchführt und anschließend Verschnitt der ein Medikament umfassenden Schicht und der Schicht aus dem ersten elastischen Film von dem getrennten Liner entfernt, wodurch auf dem getrennten Liner die bearbeitete, ein Medikament umfassende Schicht und die Schicht aus dem ersten elastischen Film in der vorbestimmten Form zurückbleiben, wodurch ein erster resultierender Streifen hergestellt wird;
- ein Klebe-Band, das ein Laminat aus einer Schicht aus einem zweiten elastischen Film, einer Klebe-Schicht und einem zweiten Schutz-Liner in dieser Reihenfolge ist, kontinuierlich von einer zweiten Zufuhr-Walze abrollt, während man den zweiten Schutz-Liner von dem Klebe-Band entfernt, und anschließend das freigemachte Klebe-Band über dem ersten resultierenden Streifen befestigt;
- einen dritten kontinuierlichen Halb-Stanz-Schritt nur an dem freigemachten Klebe-Band, das an dem ersten resultierenden Streifen befestigt ist, zu einer vorbestimmten Form in den Bereichen durchführt, die denen des zweiten Halb-Stanz-Schritts entsprechen, wobei man Verschnitt des Klebe-Bandes, der durch den dritten Halb-Stanz-Schritt erzeugt wurde, entfernt, wodurch auf dem getrennten Liner die bearbeitete, ein Medikament umfassende Schicht, die Schicht aus dem ersten elastischen Film, die Klebe-Schicht und die Schicht aus dem zweiten elastischen Film in der vorbestimmten Form zurückbleiben, wodurch ein zweiter resultierender Streifen hergestellt wird;
- einen kontinuierlichen Stanz-Schritt an dem zweiten resultierenden Streifen zu einer vorbestimmten Form in den Bereichen durchführt, die denen der Schritte des Halb-Stanzens entsprechen, wobei man Verschnitt des getrennten Liners entfernt, der durch den Schritt des Stanzens hervorgerufen wurde, wodurch man fertige medizinische Pflaster-Produkte erzeugt; und
- die fertigen medizinischen Pflaster-Produkte von der Haupt-Stanz-Walze mittels einer Vakuum-Pflaster-Walze abzieht und sie auf eine Fördereinrichtung überführt.

2. Verfahren nach Anspruch 1, worin der zweite Halb-Stanz-Schritt die ein Medikament umfassende Schicht und die Schicht des ersten elastischen Films zu einer ovalen oder runden Form ausformt und der dritte Halb-Stanz-Schritt die entsprechenden Bereiche des Klebe-Bandes zu einer ovalen oder runden Form ausformt, die geringfügig größer ist als diejenige des zweiten Halb-Stanz-Schrittes, wobei die jeweils ausgestanzten Bereiche der resultierenden Streifen durch den Stanz-Schritt so ausgeformt werden, daß sie die Bereiche jeweils des zweiten und dritten Halb-Stanz-Schrittes einschließen.

3. Verfahren nach Anspruch 1, worin der Schritt des Befestigens des freigemachten Klebe-Bandes, das auf den ersten resultierenden Streifen laminiert wird, mittels Vor- und Haupt-Andruck-Walzen in zwei Stufen durchgeführt wird.

4. Verfahren nach einem der Ansprüche 1 bis 3, worin dann, wenn das freigemachte Klebe-Band an dem ersten resultierenden Streifen befestigt wird, ein Faden oder eine Schnur ebenfalls an der Rückseite des Klebe-Bandes befestigt wird, um dem Verschnitt des Klebe-Bandes während des Aufwickelns zum Entfernen mehr Zugfestigkeit zu verleihen.

5. Vorrichtung zur Herstellung eines medizinischen Pflasters zur perkutanen Verabreichung, umfassend
- erste obere und untere Zufuhr-Walzen, wobei irgendeine der Zufuhr-Walzen einen Grund-Film abrollt und zuführt, dessen Struktur ein Laminat aus einem getrennten Liner, einer ein Medikament umfassenden Schicht, einer Schicht eines ersten elastischen Films und eines ersten Schutz-Liners in dieser Reihenfolge ist;
- eine Aufwickel-Walze für den ersten Schutz-Liner, die den ersten Schutz-Liner zum Entfernen aufwickelt;
- ein Paar erster Halb-Stanz-Walzen, die einen kontinuierlichen ersten Halb-Stanz-Schritt gerade an dem getrennten Liner des Grund-Films durchführen und so wenigstens eine wellenförmige Schneid-Reiß-Linie in Längsrichtung an dem getrennten Liner ausbilden;
- eine zweite Halb-Stanz-Walze mit einer Mehrzahl von oval geformten oder rund geformten Halb-Stanz-Eisen, die in gleichem Abstand zueinander auf deren Außenoberfläche angeordnet sind und einen kontinuierlichen zweiten Halb-Stanz-Schritt an jeder der Schichten Medikament umfassende Schicht und Schicht eines ersten elastischen Films, die auf dem getrennten Liner des Grund-Films angeordnet sind, zu einer ovalen oder runden Form ausführen;
- eine Verschnitt-Aufwickel-Walze, um die Verschnitt der ein Medikament umfassenden Schicht und der Schicht des ersten elastischen Films, der durch den zweiten Halb-Stanz-Schritt produziert wird, zum Entfernen aufgewickelt wird;
- eine zweite Zufuhr-Walze, von der ein Klebe-Band abgewickelt wird, dessen Struktur ein Laminat aus einer Schicht eines zweiten elastischen Films, einer Klebe-Schicht und einem zweiten Schutz-Liner ist;
- eine Aufwickel-Walze für den zweiten Schutz-Liner, um die der zweite Schutz-Liner zum Entfernen aufgewickelt wird;
- ein Paar Andruck-Walzen, die das freigemachte Klebeband auf den ersten resultierenden Streifen laminiert befestigen;
- eine dritte Halb-Stanz-Walze mit einer Mehrzahl von oval geformten oder rund geformten Halb-Stanz-Eisen, die in gleichem Abstand zueinander auf deren Außenoberfläche angeordnet sind und einen kontinuierlichen dritten Halb-Stanz-Schritt an Bereichen des Klebe-Bandes, das an dem ersten resultierenden Streifen befestigt ist, zu einer ovalen oder runden Form entsprechend den mit dem zweiten Halb-Stanz-Schritt gestanzten Bereichen der ein Medikament umfassenden Schicht und der Schicht des ersten elastischen Films durchführen;
- eine Walze, auf die Verschnitt des Klebe-Bandes zum Entfernen aufgewickelt wird, der durch den dritten Halb-Stanz-Schritt erzeugt wird;
- eine Stanz-Walze mit einer Mehrzahl von Stanz-Eisen, die in gleichem Abstand zueinander auf deren Außenoberfläche angeordnet sind und einen kontinuierlichen Stanz-Schritt an dem resultierenden zweiten Streifen, der den getrennten Liner, die oval oder rund ausgestanzten Schichten Medikament umfassende Schicht und Schicht des ersten elastischen Films, die oval oder rund ausgestanzten Schichten Klebe-Schicht und Schicht des zweiten elastischen Films aufweist, zu einer vorbestimmten Form durchführen und so jeden Bereich einschließen, der durch den zweiten und den dritten Halb-Stanz-Schritt gestanzt wurde;
- eine Walze, auf die Verschnitt des resultierenden Streifens zum Entfernen aufgewickelt wird, der durch den Stanz-Schritt geschaffen wird;
- eine Haupt-Stanz-Walze, die eine Mehrzahl von Vakuum-Löchern auf ihrer Außenoberfläche aufweist, zum Abziehen des getrennten Liners von dem Grund-Film und zum Durchführen des zweiten Halb-Stanz-Schrittes an der ein Medikament umfassenden Schicht und an der Schicht des ersten elastischen Films, die auf dem getrennten Liner gebildet wurde, mit Hilfe der zweiten Halb-Stanz-Walze, des dritten Halb-Stanz-Schrittes an dem freigemachten Klebe-Band mit Hilfe der dritten Halb-Stanz-Walze und des Stanz-Schrittes an dem zweiten resultierenden Streifen mit Hilfe der Stanz-Walze, wodurch fertige medizinische Pflaster-Produkte hergestellt werden; und
- eine Vakuum-Pflaster-Walze, die eine Mehrzahl von Saug-Einrichtungen aus Kautschuk auf ihrer Außenoberfläche aufweist, zum Abziehen der fertigen Produkte von der Haupt-Stanz-Walze und deren Überführung auf eine Fördereinrichtung.

6. Vorrichtung nach Anspruch 5, worin ein Paar der Andruck-Walzen zwischen der zweiten und der dritten Halb-Stanz-Walze angeordnet ist und eine der Walzen eine Vor-Andruck-Walze und die andere eine Haupt-Andruck-Walze ist.

7. Vorrichtung nach Anspruch 5 oder 6, umfassend weiter eine Einrichtung zum Aufbringen von mehr Zugfestigkeit auf den Verschnitt des Klebe-Bandes, der nach dem dritten Halb-Stanz-Schritt entfernt wird.

8. Vorrichtung nach Anspruch 7, worin die Einrichtung ein Faden oder eine Schnur ist, der/die an beiden Längskanten und/oder im Mittelabschnitt des Klebe-Bandes in Längsrichtung vorgesehen ist.

9. Vorrichtung nach Anspruch 5, umfassend weiter vorübergehend aufwickelnde Walzen, von denen jede benachbart ist der Aufwickel-Walze des Verschnitts der Medikamenten-Schicht, der Aufwickel-Walze des zweiten Schutz-Liners, bzw. der Walze zum Aufwickeln des Verschnitts des Klebe-Bandes und die nur dann vorübergehend verwendet werden, wenn die Verschnitte voll auf den drei Walzen aufgewickelt sind.

## Revendications

1. Une méthode de fabrication d'un pansement médicinal pour administration percutanée, comprenant les étapes de:
- déroutage en continu d'un film de base qui est un stratifié d'un revêtement séparé, d'une couche médicamenteuse, d'une première couche de film élastique, et d'un premier revêtement détachable, dans l'ordre, de l'un des premiers rouleaux d'alimentation tout en détachant ledit premier revêtement détachable dudit film de base;
- exécution d'un premier demi-découpage à la matrice uniquement sur le revêtement séparé en faisant passer le film de base libéré entre une paire d'un premier rouleau de demi-découpe à matrice de manière à former au moins une ligne de découpage et détachement de forme ondulée sur le revêtement séparé;
- exécution d'une seconde demi-découpe à la matrice uniquement sur la couche médicamenteuse et la première couche de film élastique disposée sur le revêtement séparé selon une forme prédéterminée et ensuite, élimination d'une chute de la couche médicamenteuse de la première couche de film élastique du revêtement séparé, par laquelle sur la couche séparée reste la couche médicamenteuse traitée et la première couche de film élastique sous la forme prédéterminée pour constituer la première bande résultante;
- déroulage en continu d'une bande adhésive qui est un stratifié d'une seconde couche de film élastique, d'une couche d'adhésif et d'un second revêtement détachable dans l'ordre d'un second rouleau d'alimentation, tout en éliminant le second revêtement détachable dudit ruban adhésif et ensuite, fixation dudit ruban adhésif libéré sur la première bande résultante;
- exécution d'une troisième demi-découpe à la matrice uniquement sur le ruban adhésif libéré qui est fixé à la première bande résultante en une forme prédéterminée aux régions correspondantes à celles de la seconde demi-découpe à la matrice tout en éliminant une chute dudit ruban adhésif engendré par la troisième demi-découpe à la matrice, de sorte que sur le revêtement séparé reste la couche médicamenteuse traitée, la première couche de film élastique, la couche d'adhésif et la seconde couche de film élastique en la forme prédéterminée pour constituer la seconde bande résultante;
- exécution d'une découpe à la matrice continue sur ladite seconde bande résultante en une forme prédéterminée aux régions correspondantes à celles desdites demi-découpes à la matrice, tout en éliminant une chute de revêtement séparé créée par ladite découpe, pour obtenir ainsi des produits finis de pansement médicinal; et
- reprise du produit fini de pansements médicinaux du rouleau principal de matrice par un rouleau à suceur sous vide et transfert de ceux-ci à un convoyeur.

2. Une méthode selon la revendication 1, dans laquelle la seconde demi-découpe forme la couche médicamenteuse et la première couche de film élastique en une forme ovale ou ronde, et la troisième demi-découpe de la matrice forme les régions correspondantes du ruban adhésif en une forme ovale ou ronde légèrement plus grande que celle de la seconde demi-découpe à la matrice, tandis que chaque région découpée des bandes résultantes sont formées par la découpe à la matrice de manière à enfermer chacune des régions obtenues par les seconde et troisième demi-découpes à la matrice.

3. Une méthode selon la revendication 1, dans laquelle l'étape de fixation du ruban adhésif libéré stratifié sur la première bande résultante est effectuée par des rouleaux presseurs préliminaires et principaux en deux étapes.

4. Une méthode selon l'une quelconque des revendications 1 à 3, dans laquelle, lorsque le ruban adhésif libéré est fixé à la première bande résultante, un filet ou cordon est également fixé au dos du ruban adhésif pour renforcer sa résistance à la traction de la chute du ruban adhésif au cours de son bobinage pour élimination.

5. Un dispositif de fabrication d'un pansement médicinal pour l'administration percutanée comprenant:
- des premiers rouleaux d'alimentation supérieurs et inférieurs, l'un ou l'autre desdits rouleaux d'alimentation déroulant et alimentant un film de base dont la structure est un stratifié d'un revêtement séparé, d'une couche médicamenteuse, d'une première couche de film élastique et d'un revêtement détachable dans l'ordre;
- un rouleau de bobinage du premier revêtement de libération qui bobine le premier revêtement de libération autour de lui pour l'élimination;
- une paire de premiers rouleaux de demi-découpe à la matrice exécutant une première demi-découpe à la matrice uniquement sur le revêtement séparé du film de base pour former au moins une ligne de découpe et de détachement de forme ondulée dans le sens de la longueur sur le revêtement séparé;
- un second rouleau de semi-découpe à la matrice ayant une pluralité de lame de demi-découpe à la matrice de forme ovale ou ronde disposé de façon équidistante sur sa surface extérieure, exécutant une seconde semi-découpe à la matrice continue sur chacune des couches médicamenteuse et premières couches de film élastique disposées sur le revêtement séparé du film de base, en une forme ovale ou ronde;
- un rouleau de reprise de chute autour duquel les chutes ou déchets de couches médicamenteuses et de premières couches de film élastique produites par la seconde demi-découpe à la matrice est enroulé pour élimination;
- un second rouleau d'alimentation d'où se déroule un ruban adhésif dont la structure est un stratifié d'une seconde couche de film élastique, d'une couche adhésive et d'un second revêtement détachable;
- un second rouleau de reprise de revêtement détachable autour duquel le second revêtement détachable s'enroule pour élimination;
- une paire de rouleau de pressage fixant le ruban adhésif libéré stratifié sur la première bande résultante;
- un troisième rouleau de demi-découpe comportant une pluralité de lames de demi-découpe de forme ovale ou ronde disposé de façon équidistante sur sa surface extérieure, exécutant une troisième demi-découpe à la matrice continue sur les régions du roulement adhésif fixé à la première bande résultante en une forme ovale ou ronde, correspond aux secondes régions de demi-découpe de la couche médicamenteuse et de la première couche de film élastique;
- un rouleau sur lequel les chutes de ruban adhésif produites par la troisième demi-découpe à la matrice est enroulé pour élimination;
- un rouleau de découpage avec une pluralité de lames de découpe disposées de façon équidistante sur sa surface extérieure, exécutant une découpe à la matrice continue d'une seconde bande résultante qui porte un revêtement séparé, les couches de forme ovale ou ronde découpées, médicamenteuses et de premiers films élastiques, les couches d'adhésifs et de seconds films élastiques découpés ovalement ou circulairement, en une forme prédéterminée, enfermant ainsi chaque région découpée dans les seconde et troisième étapes de demi-découpe à la matrice;
- un rouleau sur lequel la chute de bande résultante, créée par la découpe à la matrice, est enroulé pour élimination;
- un rouleau de matrice principale ayant une pluralité de trous à vide sur sa surface extérieure pour aspirer le revêtement séparé du film de base qui est exécuté la seconde demi-découpe à la matrice sur la couche médicamenteuse et la première couche de film élastique formée sur le revêtement séparé grâce à l'utilisation dudit second rouleau de demi-découpe à la matrice, la troisième demi-découpe à la matrice sur le ruban adhésif libéré à l'aide dudit troisième rouleau de demi-découpe à la matrice et la découpe à la matrice sur la seconde bande résultante à l'aide dudit rouleau de découpe, pour obtenir ainsi des produits finis de pansements médicaux; et
- un rouleau à suceur à vide comportant une pluralité de moyens d'aspiration de caoutchouc sur sa surface externe pour aspirer le produit fini du rouleau de découpe principal et le transférer sur un convoyeur.

6. Un dispositif selon la revendication 5, dans lequel une paire de dits rouleaux de pressage sont intercalés entre les second et troisième rouleaux de demi-découpe à la matrice et l'un d'entre eux est un rouleau préliminaire de pressage et le second étant un rouleau de pressage principal.

7. Un dispositif selon l'une quelconque des revendications 5 ou 6, comprenant en outre des moyens pour ajouter de la résistance à la traction à la chute de ruban adhésif qui est éliminé après la troisième demi-découpe à la matrice.

8. Un dispositif selon la revendication 7, dans lequel lesdits moyens est un fil ou un cordon longitudinal disposé sur les deux bords longitudinaux et/ou la partie centrale du ruban adhésif.

9. Un dispositif selon la revendication 5, comprenant en outre des rouleaux de bobinage temporaires, chacun d'eux étant adjacents audit rouleau de bobinage de la chute de médicament, ledit rouleau de bobinage de second revêtement détachable et ledit rouleau pour bobiner la chute du ruban adhésif respectivement et utilisé temporairement seulement lorsque les chutes sont totalement enroulées sur lesdits trois rouleaux.
